# EUROPEAN PATENT APPLICATION

(11) **EP 1 792 654 A2**
(43) Date of publication of application: **06.06.2007**
(21) Application number: 06019338.0
(22) Date of filing: 15.09.2006
(51) Int. Cl.: B01L 3/00, G01N 33/543

(54) **Chemical processing cartridge and method of using same**

(30) Priority: 06.10.2005 JP 2005293155
(71) Applicant: Yokogawa Electric Corporation, Tokyo 180-8750 (JP)
(72) Inventor: Tanaami, Takeo, Musashino-shi Tokyo 180-8750 (JP); Aoki, Hidetoshi, Musashino-shi Tokyo 180-8750 (JP)
(74) Representative: Goddar, Heinz J.

(57) **Abstract**

A cartridge capable of making effective use of a sample is provided. A blood sample is injected into a well (21) via an injection path (41) by use of a syringe, and so forth. A roller (6), in a state as kept pressed into contact with the cartridge, is rotated rightward, whereupon an elastic member (2) undergoes elastic deformation to cause the blood sample held in the well (21), and a liquid solvent held in a well (22) to reach a well (23), and the blood sample is thereby mixed with the liquid solvent. Since the liquid solvent contains surfactant, blood cells are destroyed in the well (23). The liquid solvent reaches a well (26) via a flow path (45). At this point in time, magnetic particles held in the well (25), and a cleaning liquid held in the well (24) are merged with the mixed liquid in the well (26). A magnet (7) is shifted from a position corresponding to the well (26) to a position corresponding to the well (31) along the flow path (46), thereby separating and transferring DNA caught by the magnetic particles to the well (31). Residue inside the well (26) after removal of DNA is transferred to a well (54) by the roller. The separated biopolymers are analyzed in the cartridge.

## Description

### FIELD OF THE INVENTION

The invention relates to a chemical processing cartridge capable of causing deformation to occur thereto upon application of an external force thereto, so as to transfer, or seal substances contained therein, thereby causing a chemical process to proceed, and a method of using the same.

### BACKGROUND OF THE INVENTION

The chemical reaction cartridge that can cause a chemical reaction to proceed by transferring, or sealing substances contained therein, due to deformation occurring thereto, upon application of an external force thereto, has been under development.
[Patent Document 1] JP 2004 - 226068 A

### SUMMARY OF THE INVENTION

Specific DNA sequences and proteins are used as disease markers in clinical diagnosis. The disease marker can be extracted from a blood sample and so forth. However, one type of protein is normally detected at a time by the ELIS (EMA) method, and so forth. Normally, for detection of DNA markers, a blood sample for DNA extraction is taken separately from a blood sample for detection of protein markers.

Accordingly, in order to make a diagnosis at a clinical test, samples of blood and so forth must be taken from a patient for every diagnostic item. In order to make a reliable diagnosis, tests on a plurality of markers, respectively, are required, so that there will be an increase in the number of samples to be taken, resulting in an increase in burden imposed on the patient. Further, since only a sample small in quantity is obtained by biopsy, it is difficult to use such a sample for a multitude of test items. In order to avoid noises at the time of detection, it is a practice at present to discard DNA fractions at the time of protein detection, and to protein fractions at the time of DNA detection. However, it is naturally more desirable to detect both the fractions from the same sample from the viewpoint of full utilization of a sample quantity, and identity of the sample used for the tests. Although a thought occurs to use of an autoanalyzer (a fully-automatic test machine) as a method for utilizing the same sample for a multitude of test items, the autoanalyzer is not only large in size and expensive but also requires countermeasures against virus since detection in an open system is executed, so that it is difficult for a common hospital to make use of the autoanalyzer.

It is an object of the invention to provide a cartridge capable of making effective use of a sample by utilizing the technology of the chemical reaction cartridge as disclosed in JP 2004 - 226068 A, referred to as above.

In accordance with a first aspect of the invention, there is provided a chemical processing cartridge capable of causing deformation to occur thereto upon application of an external force thereto, and transferring or sealing substances contained therein, thereby causing a chemical process to proceed, said cartridge comprising a receiving means for receiving a sample from outside, a separation means for executing separation between a plurality of biopolymers of different species, contained in the sample received from the outside, and at least two analysis means for analyzing the respective biopolymers as separated.

With the chemical processing cartridge described, the plurality of the biopolymers of the different species, contained in the sample received from the outside, can be separated from each other, so that it is possible to make effective use of the sample, and to ensure identity of the sample as a target for the analysis. With the chemical processing cartridge according to the present invention, there is no limitation to types of the sample.

Further, since the chemical processing cartridge is provided with the analysis means for analyzing the respective biopolymers as separated, it is possible to analyze the respective biopolymers as separated within the cartridge.

For the separation means, use may be made of any selected from the group consisting of silica beads, styrene beads, glass fiber, and cellulose.

For the separation means, use may be made of magnetic particles.

The biopolymers may be any selected from the group consisting of DNA, RNA, proteins, sugar chains, and metabolites.

The sample may be a body fluid.

The plurality of the biopolymers of the different species may be each a marker common to respective diseases.

In such a case, a diversified analysis can be carried out with respect to the respective diseases. Further, if the cartridge is made up in such a way as to separate between the respective markers for a multitude of diseases, or analyze the same, this will be effective for screening the respective diseases.

The biopolymers may be at least one species of any biopolymer selected from the group consisting of biopolymers related to various diseases, respectively, shown as follows:
(a) AFP, PIVK-II, isocitrate dehydrogenase, and YH-206, in the case of hepatoma;
(b) CA125, NCC-ST- 439, STN (serial Tn antigen) CEA, CA72-4, and CA19-9, in the case of gastric cancer;
(c) CA19-9, YH-206, NCC-ST-439, CA19-9, CA50, Span-1, DUPAN-2, CEA, and SLX, in the case of pancreatic cancer;
(d) CEA, SCC, and CYFRA21-1, in the case of esophagus cancer;
(e) SCC, CYFRA21-1, SLX, CEA, NSE, and Pro-GRP, in the case of lung cancer;
(f) BFP, in the case of kidney cancer;
(g) CA15-3 (MAN-6), CEA, and NCC-ST-439, in the case of breast cancer;
(h) ErbB-2 (Her2), and BRCA1 gene, in the case of breast cancer;
(i) CA125, CA72-4, STN, and GAT, in the case of ovary cancer;
(j) PSA, and γ-Sm (γ-seminoprotein), in the case of prostate cancer;
(k) adeponecutin, TNF-α, PA1-1, glicosylated hemoglobin, A1c (HbA1c), and CPR (proinsulin), in the case of diabetes;
(l) myoglobin, H-FABP, CK-MB, and troponin T, in the case of myocardial infarct;
(m) HBs antigen, HBe antigen, and HCV antigen, in the case of hepatitis;
(n) the intrinsic gene of a virus or bacterium, and an antibody against these pathogens.

The invention provides in its second aspect a chemical processing cartridge capable of causing deformation to occur thereto upon application of an external force thereto, and transferring or sealing substances contained therein, thereby causing a chemical process to proceed, said cartridge comprising a receiving means for receiving a sample from outside, and a separation means for executing separation between a plurality of biopolymers of different species, contained in the sample received from the outside, wherein magnetic particles are used for the separation means, and a magnet is externally moved along flow paths, thereby causing the magnetic particles attracted by the magnet to be transferred along the flow paths.

With the chemical processing cartridge described, biopolymers captured by the magnetic particles can be transferred.

Further, in accordance with a third aspect of the invention, there is provided a method of using a chemical processing cartridge capable of causing deformation to occur thereto upon application of an external force thereto, and transferring or sealing substances contained therein, thereby causing a chemical process to proceed, the cartridge comprising a receiving means for receiving a sample from outside, a separation means for executing separation between a plurality of biopolymers of different species, contained in the sample received from the outside, and at least two analysis means for analyzing the respective biopolymers as separated, said method comprising the steps of injecting the sample into the receiving means, executing the separation between the biopolymers serving as markers for specific diseases with the use of the separation means, and analyzing the respective markers as separated with the use of the analysis means.

With the method of using the chemical processing cartridge, since the separation between the biopolymers serving as the markers for the specific diseases, respectively, is executed with the use of the separation means, it is possible to make effective use of the sample, and to ensure identity of the sample as a target for the analysis. Further, a diversified analysis can be carried out with respect to the respective diseases.

The method of using the chemical processing cartridge may further comprise the step of discarding the cartridge after the step of executing the separation between the markers, or the step of analyzing the respective markers.

In this case, operations such as post-treatment, cleaning, and so forth are not required while ensuring safety.

With the method of using the chemical processing cartridge, according to the invention, the biopolymers may be at least one species of any biopolymer selected from the group consisting of biopolymers related to various diseases, respectively, shown as follows:
(a) AFP, PIVK-II, isocitrate dehydrogenase, and YH-206, in the case of hepatoma;
(b) CA125, NCC-ST- 439, STN (serial Tn antigen) CEA, CA72-4, and CA19-9, in the case of gastric cancer;
(c) CA19-9, YH-206, NCC-ST-439, CA19-9, CA50, Span-1, DUPAN-2, CEA, and SLX, in the case of pancreatic cancer;
(d) CEA, SCC, and CYFRA21-1, in the case of esophagus cancer;
(e) SCC, CYFRA21-1, SLX, CEA, NSE, and Pro-GRP, in the case of lung cancer;
(f) BFP, in the case of kidney cancer;
(g) CA15-3 (MAN-6), CEA, and NCC-ST-439, in the case of breast cancer;
(h) ErbB-2 (Her2), and BRCA1 gene, in the case of breast cancer;
(i) CA125, CA72-4, STN, and GAT, in the case of ovary cancer;
(j) PSA, and γ-Sm (γ-seminoprotein), in the case of prostate cancer;
(k) adeponecutin, TNF-α, PA1-1, glicosylated hemoglobin, A1c (HbA1c), and CPR (proinsulin), in the case of diabetes;
(l) myoglobin, H-FABP, CK-MB, and troponin T, in the case of myocardial infarct;
(m) HBs antigen, HBe antigen, and HCV antigen, in the case of hepatitis;
(n) the intrinsic gene of a virus or bacterium, and an antibody against these pathogens.

With the chemical processing cartridge according to the invention, since the plurality of the biopolymers of the different species, contained in the sample received from the outside, can be separated from each other, it is possible to make effective use of the sample, and to ensure the identity of the sample as the target for the analysis.

With the method of using the chemical processing cartridge, according to the invention, since the separation between the biopolymers serving as the markers for the specific diseases, respectively, is executed with the use of the separation means, it is possible to make effective use of the sample, and to ensure the identity of the sample as the target for the analysis. Further, the diversified analysis can be carried out with respect to the respective diseases.

### BRIEF DESCRIPTION OF THE DRAWINGS

Fig. 1 is a view showing a construction of a chemical reaction cartridge according to Embodiment 1 of the invention, in which Fig. 1(A) is a plan view of the cartridge, and Fig. 1(B) is a sectional view showing a section of the cartridge, taken along wells and flow paths in Fig. 1(A);
Fig. 2 is a view showing a construction of a chemical reaction cartridge according to Embodiment 2 of the invention, in which Fig. 2(A) is a plan view of the cartridge, and Fig. 2(B) is a sectional view showing a section of the cartridge, taken along wells and flow paths in Fig. 2(A); and
Fig. 3 is a view showing proteins, sugar chains, and DNA, serving as targets in tests for varieties of diseases, respectively, by way of example.

### PREFERRED EMBODIMENTS OF THE INVENTION

Embodiments of a chemical processing cartridge according to the invention are described hereinafter.

### Embodiment 1

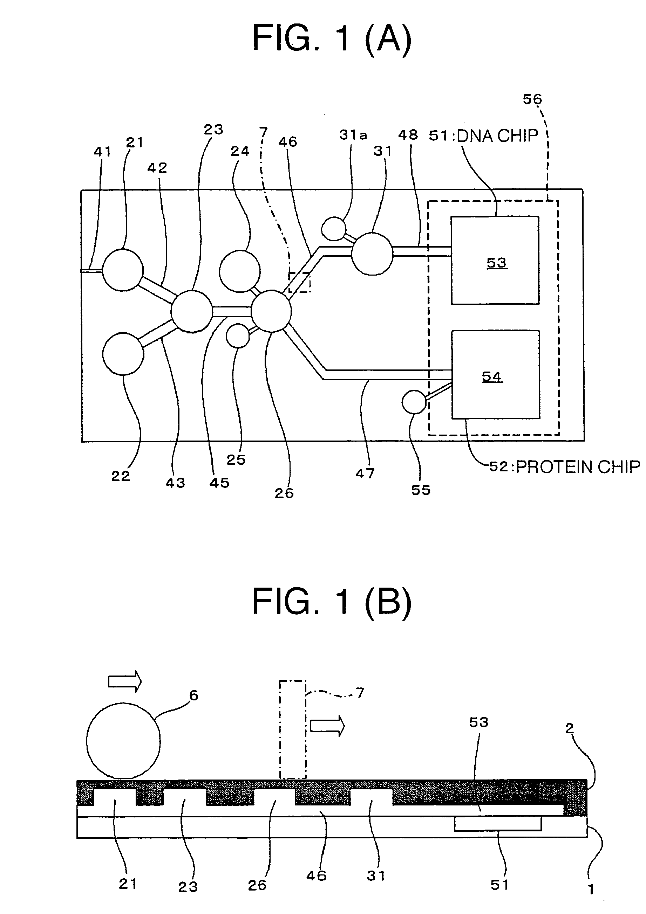

A chemical processing cartridge according to Embodiment 1 of the invention is described hereinafter with reference to Fig. 1.

The present embodiment represents a cartridge for concurrently analyzing DNA and protein on the basis of a single sample.

Fig. 1(A) is a plan view of the cartridge according to the present embodiment, and Fig. 1(B) is a sectional view showing the cartridge in section, taken along wells and flow paths shown in Fig. 1(A).

As shown in Fig. 1(B), a vessel of the cartridge according to the present embodiment comprises a substrate 1, and an elastic member 2 overlaid on the substrate 1.

Recesses, each in a predetermined shape, depressed toward the top surface of the elastic member 2 (the upper surface thereof, in Fig. 1(B)) are formed in the back surface of the elastic member 2 (the underside surface thereof, in Fig. 1(B)). The recesses create space between the substrate 1, and the elastic member 2, and as shown in Figs. 1(A), 1(B), there are formed a well 21 for receiving a sample, a well 22 for pre-holding a liquid solvent, a well 23 for mixing the sample with the liquid solvent, a well 24 for pre-holding a cleaning liquid, a well 25 for holding magnetic particles together with a flowing liquid, a well 26 for mixing the sample, cleaning liquid, and magnetic particles together, a well 31 for executing PCR amplification, a well 31a connected to the well 31, an injection path 41 for injecting the sample into the well 21, a flow path 42 for connecting the well 21 to the well 23, a flow path 43 for connecting the well 22 to the well 23, a flow path 45 for connecting the well 23 to the well 26, a flow path 46 for connecting the well 26 to the well 31, a flow path 47 connected to the well 26, and a flow path 48 connected to the well 31.

As shown in Figs. 1(A), 1(B), a DNA chip 51 for DNA analysis, and a protein chip 52 for protein analysis are embedded in the substrate 1. There are formed a well 53 in a recess of the elastic member 2, on the top surface side of the DNA chip 51 (the upper surface thereof, in Fig. 1(B)), and a well 54 in a recess of the elastic member 2, on the top surface side of the protein chip 52 (the upper surface thereof, in Fig. 1(B)), respectively, and the flow path 48 is connected to the well 53 while the flow path 47 is connected to the well 54, respectively. Further, a well 55 for holding secondary antibodies together with the flowing liquid is connected to the well 54.

Next, there is described hereinafter an analysis method using the cartridge according to the present embodiment.

First, a blood sample is injected into the well 21 via the injection path 41 by use of a syringe, and so forth.

Then, as shown in Fig. 1(B), a roller 6, in a state as kept pressed into contact with the cartridge, is rotated rightward, whereupon the elastic member 2 undergoes elastic deformation to cause the blood sample held in the well 21, and the liquid solvent held in the well 22 to reach the well 23 via the flow paths 42, 43, respectively, and the blood sample is thereby mixed with the liquid solvent. Since the liquid solvent contains surfactant, blood cells are destroyed in the well 23.

Upon further rotation of the roller 6, a mixed liquid reaches the well 26 via the flow path 45. At this point in time, the magnetic particles held in the well 25, and the cleaning liquid held in the well 24 are merged with the mixed liquid in the well 26.

Subsequently, the roller 6 is stopped, and as shown in Figs. 1(A), 1(B), a magnet 7 is shifted from a position corresponding to the well 26 to a position corresponding to the well 31 along the flow path 46, thereby transferring DNA caught by the magnetic particles to the well 31. The magnetic particles are selected as appropriate among particles in general, having the capability of adsorbing nucleic acid, and collecting nucleic acid as-bonded state by the agency of magnetism.

Next, rotation of the roller 6 is resumed, and residue inside the well 26 after removal of DNA is transferred to the flow path 47. Further, at this point in time, a reagent pre-held in the well 31a is transferred to the well 31. The flow path 46 is sealed by a sealing means at the time when the residue is transferred to the flow path 47. As the sealing means, the flow path 46 may be sealed by, for example, pressing down the magnet 7 onto the cartridge. In this connection, a transfer path of the roller 6, and a sealing position of the sealing means are appropriately controlled so as to be positioned, respectively, in such a way as to prevent occurrence of mutual contact therebetween. Otherwise, transfer of substances after the well 26 may be effected on a flow-path-by-flow-path basis by use of other rollers (not shown).

The reagent in the well 31a contains a mixed liquid of primer and deoxyribonucleotide, and DNA synthetic enzyme, and PCR amplification of DNA is executed in the well 31 by controlling temperature of the well 31. The temperature of the well 31 is controlled by an apparatus using, for example, a heater or a Peltier element.

Then, upon further rotation of the roller 6, PCR byproducts produced in the well 31 are transferred to the well 53 via the flow path 48. Further, at this point in time, the residue in the flow path 47 is transferred to the well 54, whereupon the secondary antibodies in the well 55 are merged with the residue in the well 54.

DNA in the PCR byproducts transferred to the well 53 is bonded to a specific probe of the DNA chip, so that DNA analysis by fluorescence can be executed.

Further, proteins in the residue transferred to the well 54 is captured by the protein chip 52 due to an antigen-antibody reaction, so that protein analysis as well as DNA analysis can be executed. The DNA analysis, and the protein analysis are enabled by irradiation with excitation light, and capturing of fluorescence, through the intermediary of constituent members of the cartridge, without taking the DNA chip 51, and the protein chip 52 out of the cartridge.

Upon execution of the DNA analysis, and the protein analysis, necessary images can be concurrently captured by picking up, for example, image of a region 56 (Fig. 1(A)) in the cartridge by use a camera.

With the embodiment described as above, tests for a plurality of proteins, and DNA, respectively, can be concurrently conducted on the same sample. Accordingly, a small usage of a precious sample is sufficient for the purpose of the tests, thereby lightening a burden imposed on a patient.

Further, since the identity of the sample is ensured, reliability of the tests can be enhanced.

Furthermore, test results are found in short time, and analysis time is significantly shortened. In addition, automation of the tests can be implemented with ease, and since neither a special skill nor a special device is required for handling the cartridge, material as well as personal cost of the tests can be lightened.

Still further, with the use of the cartridge according to the embodiment described as above, a DNA test can be rapidly conducted. In general, in the case of a test of DNA on the basis of a blood sample, and so forth, with a sample in which solidification constituents are precipitated by the agency of sodium citrate, EDTA, or heparin, a PCR reaction necessary for SNPs is blocked. For this reason, it is required that for DNA detection, fresh blood or rapidly frozen blood is used, and those skilled in operation are to handle the sample. With the cartridge according to the present invention, since a predetermined processing can be rapidly executed by a simple operation, it becomes possible to implement stable DNA detection without operational burdens.

### Embodiment 2

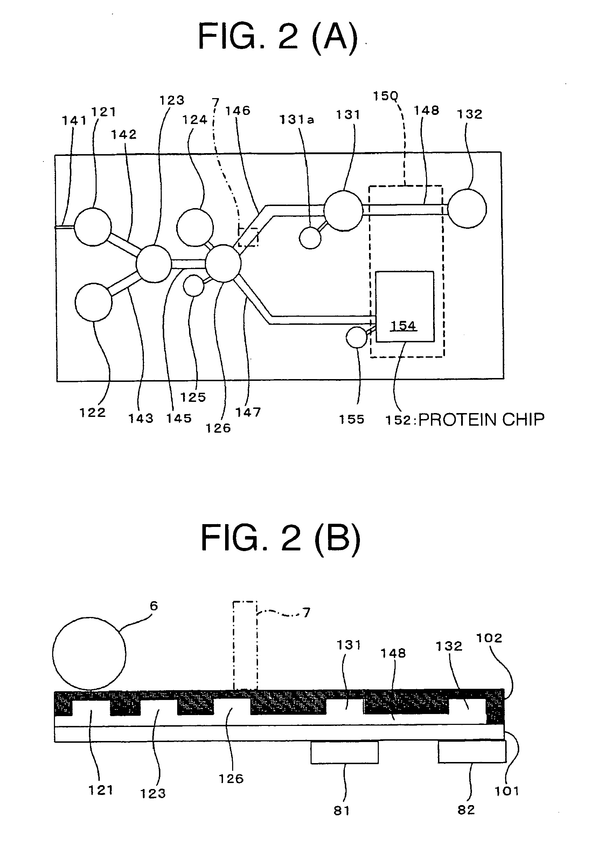

A chemical processing cartridge according to Embodiment 2 of the invention is described hereinafter with reference to Fig. 2. The present embodiment represents a cartridge for concurrently detecting gene variations {SNPs (single nucleotide polymorphisms)} associated with specific diseases, and respective proteins related to the gene variations.

For a gene analysis, use is made of a real-time PCR method. The real-time PCR method is a method for monitoring an amount of DNA amplification by PCR in real time to thereby execute an analysis, requiring no electrophoresis, and excellent in rapidity and quantification. With the method, a temperature cycle under a given condition is applied to a sample of DNA in unknown concentration to cause PCR amplification to proceed, thereby finding the number of cycles until a given amount of an amplification product is obtained. If there is prepared in advance a calibration curve indicating the number of cycles until an amount of an amplification product of a known amount of DNA, obtained dilution by stages, reaches the same amount in the same condition, this will enable an amount of the DNA in the sample to be measured on the basis of the calibration curve

Fig. 2(A) is a plan view of the cartridge according to the present embodiment, and Fig. 2(B) is a sectional view showing the cartridge in section, taken along wells and flow paths shown in Fig. 2(A).

As shown in Fig. 2(B), a vessel of the cartridge according to the present embodiment comprises a substrate 101, and an elastic member 102 overlaid on the substrate 101.

Recesses, each in a predetermined shape, depressed toward the top surface of the elastic member 102 (the upper surface thereof, in Fig. 2(B)) are formed in the back surface of the elastic member 102 (the underside surface thereof, in Fig. 2(B)). The recesses create space between the substrate 101, and the elastic member 102, and as shown in Figs. 2(A), 2(B), there are formed a well 121 for receiving a sample, a well 122 for pre-holding a liquid solvent, a well 123 for mixing the sample with the liquid solvent, a well 124 for pre-holding a cleaning liquid, a well 125 for holding magnetic particles together with a flowing liquid, a well 126 for mixing the sample, cleaning liquid, and magnetic particles together, a well 131 and a well 132 for executing PCR amplification, a well 131a connected to the well 131, an injection paths 141 for injecting the sample into the well 121, a flow path 142 for connecting the well 121 to the well 123, a flow path 143 for connecting the well 122 to the well 123, a flow path 145 for connecting the well 123 to the well 126, a flow path 146 for connecting the well 126 to the well 131, a flow path 147 connected to the well 126, and a flow path 148 for connecting the well 131 to a well 132.

As shown in Figs. 2(A), 2(B), a protein chip 152 for protein analysis is embedded in the substrate 101. Further, there is formed a well 154 in a recess of the elastic member 102, on the top surface side of the protein chip 152, and the flow path 147 is connected to the well 154. Further, a well 155 for holding secondary antibodies together with the flowing liquid is connected to the well 154.

Next, there is described hereinafter an analysis method using the cartridge according to the present embodiment.

First, a blood sample is injected into the well 121 via the injection path 141 by use of a syringe, and so forth.

Then, as shown in Fig. 2(B), a roller 6, in a state as kept pressed into contact with the cartridge, is rotated rightward, whereupon the elastic member 102 undergoes elastic deformation to cause the blood sample held in the well 121, and the liquid solvent held in the well 122 to reach the well 123 via the flow paths 142, 143, respectively, and the blood sample is thereby mixed with the liquid solvent. Since the liquid solvent contains surfactant, blood cells are destroyed in the well 123.

Upon further rotation of the roller 6, a mixed liquid reaches the well 126 via the flow path 145. At this point in time, the magnetic particles held in the well 125, and the cleaning liquid held in the well 124 are merged with the mixed liquid in the well 126.

Subsequently, the roller 6 is stopped, and as shown in Figs. 2(A), 2(B), a magnet 7 is shifted from a position corresponding to the well 126 to a position corresponding to the well 131 along the flow path 146, thereby transferring DNA caught by the magnetic particles to the well 131.

Next, rotation of the roller 6 is resumed, and residue inside the well 126 after removal of DNA is transferred to the well 154 via the flow path 147, whereupon the secondary antibodies in the well 155 is merged with the residue inside the well 154. Further, at this point in time, a reagent pre-held in the well 131a is transferred to the well 131. The flow path 146 is sealed by a sealing means at the time when the residue is transferred to the flow path 147. As the sealing means, the flow path 146 may be sealed by, for example, pressing down the magnet 7 onto the cartridge. In this connection, a transfer path of the roller 6, and a sealing position of the sealing means are appropriately controlled so as to be positioned, respectively, in such a way as to prevent occurrence of mutual contact therebetween. Otherwise, transfer of substances after the well 126 may be effected on a flow-path-by-flow-path basis by use of other rollers (not shown).

Further, proteins in the residue transferred to the well 154 can be captured by the protein chip 152 due to an antigen-antibody reaction, and a protein analysis is executed at an appropriate time. Upon the protein analysis, for example, by irradiation of the cartridge with excitation light from outside, and by photographing a region of the protein chip 152 with a camera, necessary images can be captured without taking the protein chip 152 out of the cartridge.

Meanwhile, the reagent in the well 131a contains a mixed liquid of primer and deoxyribonucleotide, DNA synthetic enzyme, and a real-time detection probe, and in the well 131, PCR amplification of specific DNA is started. As a method for adding the detection probe, there is known, for example, an intercalater method. With this method, an intercalater {for example, SYBR (trade mark) Green 1} emitting fluorescence upon binding to duplex DNA is used as the detection probe. The intercalater is bonded to the duplex DNA synthesized by PCR reaction and emits fluorescent light by irradiation with excitation light. By detection of intensity of the fluorescent light, it is possible to monitor a production amount of an amplification product.

As shown in Fig. 2(B), the well 131, and the well 132 are controlled to respective given temperatures (for example, on the order of 60°C, 95°C) by temperature controllers 81, 82, using, for example, a heater or a Peltier element, respectively.

With the present embodiment, by driving the roller 6, respective PCR byproducts are caused to reciprocate between the well 131 and the well 132 according to a given cycle.

An amplification production amount of the PCR byproducts reciprocating between those wells is measured in real time based on a quantity of fluorescent light by photographing the flow path 148 with a camera while irradiating the same with excitation light. In the case where a region 150 shown in Fig. 2(B) is photographed with the camera, both DNA and protein can be analyzed on the basis of a single image of the region.

With the embodiment described as above, tests for proteins as a plurality of disease markers, and gene variations related to the diseases, respectively, can be concurrently conducted on the same sample. Accordingly, a small usage of a precious sample is sufficient for the purpose of the tests, thereby lightening a burden imposed on a patient.

Further, since identity of the sample is ensured, reliability of the tests can be enhanced.

Furthermore, test results are found in short time, and analysis time is significantly shortened. In addition, automation of the tests can be implemented with ease, and since neither a special skill nor a special device is required for handling the cartridge, material as well as personal cost of the tests can be lightened.

With the embodiment described as above, an analysis on one type of DNA is executed, however, the number of targets for the analysis can be optionally selected.

The cartridge according to the present invention can be used for a test on a specific disease. In the case of, for example, a blood test for checking a possibility of a breast tumor, a diversified test can be carried out through detection of four types of tumor marker proteins, and ERRB gene variations.

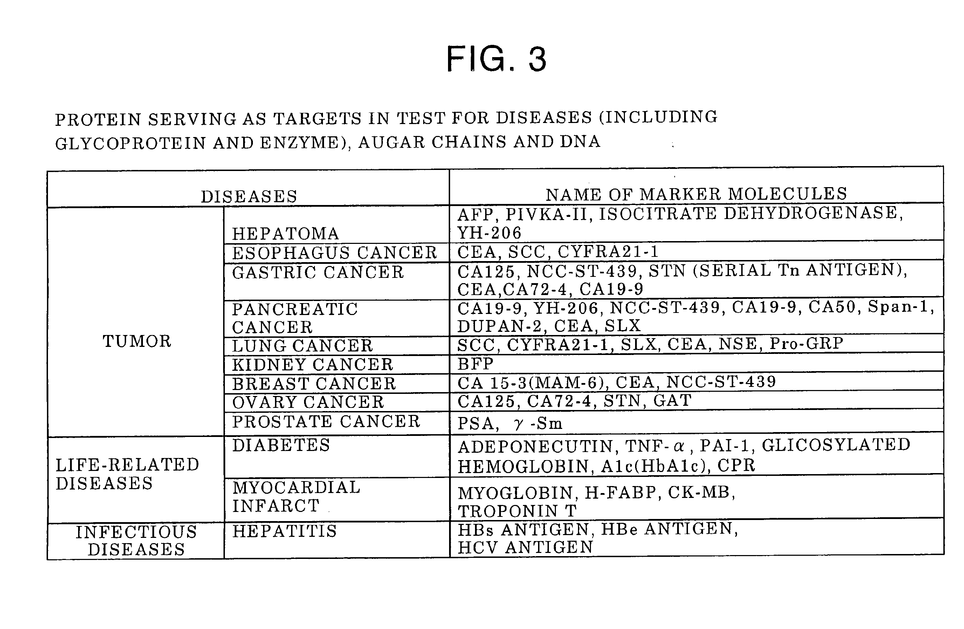

In Fig. 3, there are shown proteins, sugar chains, and DNA, serving as targets in tests for varieties of diseases, respecyivbely, by way of example.

The cartridge according to the present invention is in extensive application for respective tests of diseases such as life-related diseases including tumors of respective organs. Test items are not limited to combination of the SNPs (single nucleotide polymorphisms), and a protein, and it is also possible to detect a plurality of SNPs on a gene, and to detect a tumor marker gene increase in blood.

A makeup of the cartridge can be selected as appropriate corresponding to test content. With the embodiment described as above, DNA and a protein are detected by incorporating the DNA chip or the protein chip inside the cartridge, however, processing until the antigen-antibody reaction is executed within the cartridge, and detection of the protein may be implemented in the course of capillary electrophoresis. In such a case, the cartridge can be provided with a discharge path for transferring the sample to a detector utilizing the capillary electrophoresis. Further, in this case, in connection with protein detection, by providing an appropriate number of wells for causing to undergo the antigen-antibody reaction, it is possible to make up the cartridge capable of detecting proteins of, for example, up to about ten varieties, corresponding to the number of the wells.

Further, by pre-checking mobility of each of proteins as-bonded to respective antibodies, a variety of the proteins may be portioned out among the respective wells in such a way as to prevent overlapping of peaks of the respective mobilities, as detected by the capillary electrophoresis. In this case, the number of varieties of the antibodies that are caused to undergo reaction in the respective wells can be increased to 2 or 3, so that the number of marker proteins detectable in one assay can be rendered greater than the number of the wells.

A method of separating a protein from DNA can be selected as appropriate. For example, any selected from the group consisting of silica beads, styrene beads, glass fiber, cellulose, and so forth may be made in place of the magnetic particles by taking advantage of charge-carrying properties thereof.

With the embodiment described as above, the case of separating the protein from the DNA is shown by way of example, but it is also possible to separate DNA from mRNA by use of beads with polyaniline chain added thereto.

Further, with the embodiment described as above, the protein analysis is described by way of example, however, by use of adsorbates for sugar chain and a metabolite, respectively, separation of the sugar chain from the metabolite, or concurrent analysis thereof may be carried out inside the cartridge. For example, a protein bonded to sugar chain can be utilized as an adsorbate for the sugar chain.

As shown in Fig. 3, in the case of a pathogen, such as a virus, bacterium, and so forth, causing a disease, DNA, RNA, or a protein of the pathogen itself, or an immune antibody produced against the pathogen, and so forth may be separated or detected as a marker for the disease. It is also possible to make up, for example, such a cartridge as is capable of detecting a set of the DNA of the pathogen, and the immune antibody produced against the pathogen.

More specifically, tests for various diseases are conducted as follows.
- the case of tumor (cancer) tests by immunochemical techniques, in the case of hepatoma:
   test on AFP, PIVK-II, isocitrate dehydrogenase, YH-206; in the case of gastric cancer:
   test on CA125, NCC-ST-439, STN (serial Tn antigen) CEA CA72-4, CA19-9;
   in the case of pancreatic cancer:
   test on CA19-9, YH-206, NCC-ST-439, CA19-9, CA50, Span-1, DUPAN-2, CEA, SLX;
   in the case of esophagus cancer:
   test on CEA, SCC, CYFRA21-1;
   in the case of lung cancer:
   test on SCC, CYFRA21-1, SLX, CEA, NSE. Pro-GRP:
   in the case of kidney cancer:
   test on BFP;
   in the case of breast cancer:
   test on CA15-3 (MAN-6), CEA, NCC-ST-439, or ErbB-2 (Her2) and BRCA1 gene;
   in the case of ovary cancer:
   test on CA125, CA72-4, STN, GAT;
   in the case of prostate cancer:
   test on PSA, γ-Sm (γ-seminoprotein)
- tests for life-related diseases:
   test on adeponecutin, TNF-α, PA1-1, glicosylated hemoglobin, A1c (HbA1c), CPR (proinsulin), in the case of diabetes:
   test on myoglobin, H-FABP, CK-MB, troponin T, in the case of myocardial infarct;
- tests for infectious diseases:
   in the case of hepatitis
   test on HBs antigen, HBe antigen, HCV antigen or concurrent measurement test on the intrinsic gene of a pathogen of a virus or bacterium, and an antibody against the pathogen

With reference to the various diseases described in the foregoing, a test is conducted on a level of manifestation of respective genes or markers thereof, or/and on respective gene polymorphisms.

With the cartridge according to the present invention, biopolymers as targets for separation or analysis can include DNA, RNA, proteins, sugar chains, metabolites, and so forth

It is to be pointed that the invention is not limited in scope of application to those embodiments described hereinbefore, and that the invention is widely applicable to a chemical processing cartridge capable of causing deformation to occur thereto upon application of an external force thereto, and transferring substances contained therein, to thereby cause a chemical process to proceed, and a method for using the same.

## Claims

1. A chemical processing cartridge capable of causing deformation to occur thereto upon application of an external force thereto, and transferring or sealing substances contained therein, thereby causing a chemical process to proceed, said cartridge comprising:
a receiving means for receiving a sample from outside;
a separation means for executing separation between a plurality of biopolymers of different species, contained in the sample received from the outside; and
at least two analysis means for analyzing the respective biopolymers as separated.
biopolymers as separated within the cartridge.

2. A chemical processing cartridge according to Claim 1,
wherein for the separation means, use is made of magnetic particles.

3. A chemical processing cartridge according to Claim 1,
wherein for the separation means, use is made of any selected from the group consisting of silica beads, styrene beads, glass fiber, and cellulose.

4. A chemical processing cartridge according to Claim 1,
wherein the biopolymers is any selected from the group consisting of DNA, RNA, proteins, sugar chains, and metabolites.

5. A chemical processing cartridge according to Claim 1,
wherein the sample is a body fluid.

6. A chemical processing cartridge according to Claim 1,
wherein the plurality of the biopolymers of the different species are each a marker common to respective diseases.

7. A chemical processing cartridge according to Claim 1,
wherein the biopolymers are at least one species of any biopolymer selected from the group consisting of biopolymers related to various diseases, respectively, shown as follows:
(a) AFP, PIVK-II, isocitrate dehydrogenase, and YH-206, in the case of hepatoma;
(b) CA125, NCC-ST- 439, STN (serial Tn antigen) CEA, CA72-4, and CA19-9, in the case of gastric cancer;
(c) CA19-9, YH-206, NCC-ST-439, CA19-9, CA50, Span-1, DUPAN-2, CEA, and SLX, in the case of pancreatic cancer;
(d) CEA, SCC, and CYFRA21-1, in the case of esophagus cancer;
(e) SCC, CYFRA21-1, SLX, CEA, NSE, and Pro-GRP, in the case of lung cancer;
(f) BFP, in the case of kidney cancer;
(g) CA15-3 (MAN-6), CEA, and NCC-ST-439, in the case of breast cancer;
(h) ErbB-2 (Her2), and BRCA1 gene, in the case of breast cancer;
(i) CA125, CA72-4, STN, and GAT, in the case of ovary cancer;
(j) PSA, and γ-Sm (γ-seminoprotein), in the case of prostate cancer;
(k) adeponecutin, TNF-α, PA1-1, glicosylated hemoglobin, A1c (HbA1c), and CPR (proinsulin), in the case of diabetes;
(l) myoglobin, H-FABP, CK-MB, and troponin T, in the case of myocardial infarct;
(m) HBs antigen, HBe antigen, and HCV antigen, in the case of hepatitis;
(n) or the intrinsic gene of a pathogen of a virus or bacterium, and an antibody against the pathogen.

8. A chemical processing cartridge capable of causing deformation to occur thereto upon application of an external force thereto, and transferring or sealing substances contained therein, thereby causing a chemical process to proceed, said cartridge comprising:
a receiving means for receiving a sample from outside; and
a separation means for executing separation between a plurality of biopolymers of different species, contained in the sample received from the outside;
wherein magnetic particles are used for the separation means, and a magnet is externally moved along flow paths (46, 146), thereby causing the magnetic particles attracted by the magnet to be transferred along the flow paths (46, 146).

9. A method of using a chemical processing cartridge capable of causing deformation to occur thereto upon application of an external force thereto, and transferring or sealing substances contained therein, thereby causing a chemical process to proceed: said cartridge comprising:
a receiving means for receiving a sample from outside;
a separation means for executing separation between a plurality of biopolymers of different species, contained in the sample received from the outside; and
at least two analysis means for analyzing the respective biopolymers as separated;
said method comprising the steps of:
injecting the sample into the receiving means;
executing the separation between the biopolymers serving as markers for specific diseases with the use of the separation means; and
analyzing the respective markers as separated with the use of the analysis means.

10. A method of using the chemical processing cartridge according to Claim 9, further comprising the step of discarding the cartridge after the step of executing the separation between the markers, or the step of analyzing the respective markers.

11. A method of using the chemical processing cartridge according to Claim 9, wherein the biopolymers are at least one species of any biopolymer selected from the group consisting of biopolymers related to various diseases, respectively, shown as follows:
(a) AFP, PIVK-II, isocitrate dehydrogenase, and YH-206, in the case of hepatoma;
(b) CA125, NCC-ST- 439, STN (serial Tn antigen) CEA, CA72-4, and CA19-9, in the case of gastric cancer;
(c) CA19-9, YH-206, NCC-ST-439, CA19-9, CA50, Span-1, DUPAN-2, CEA, and SLX, in the case of pancreatic cancer;
(d) CEA, SCC, and CYFRA21-1, in the case of esophagus cancer;
(e) SCC, CYFRA21-1, SLX, CEA, NSE, and Pro-GRP, in the case of lung cancer;
(f) BFP, in the case of kidney cancer;
(g) CA15-3 (MAN-6), CEA, and NCC-ST-439, in the case of breast cancer;
(h) ErbB-2 (Her2), and BRCA1 gene, in the case of breast cancer;
(i) CA125, CA72-4, STN, and GAT, in the case of ovary cancer;
(j) PSA, and γ-Sm (γ-seminoprotein), in the case of prostate cancer;
(k) adeponecutin, TNF-α, PA1-1, glicosylated hemoglobin, A1c (HbA1c), and CPR (proinsulin), in the case of diabetes;
(l) myoglobin, H-FABP, CK-MB, and troponin T, in the case of myocardial infarct;
(m) HBs antigen, HBe antigen, and HCV antigen, in the case of hepatitis;
(n) or the intrinsic gene of a pathogen of a virus or bacterium, and an antibody against the pathogen.
